Europäisches Patentamt

European Patent Office

Office européen des brevets

(11) Publication number: **0 350 273 B1**

# EUROPEAN PATENT SPECIFICATION

(45) Date of publication of patent specification: **13.07.94**  (51) Int. Cl.5: **G01N 33/579**, G01N 33/49

(21) Application number: **89306795.9**

(22) Date of filing: **04.07.89**

The file contains technical information submitted after the application was filed and not included in this specification

(54) **Process for measuring endotoxin.**

(30) Priority: **05.07.88 JP 167068/88**

(43) Date of publication of application:
**10.01.90 Bulletin 90/02**

(45) Publication of the grant of the patent:
**13.07.94 Bulletin 94/28**

(84) Designated Contracting States:
**AT BE CH DE ES FR GB GR IT LI LU NL SE**

(56) References cited:
**EP-A- 0 173 021**
**DE-A- 2 517 860**
**JP-A-62 110 153**
**US-A- 4 038 029**

(73) Proprietor: **WAKO PURE CHEMICAL INDUS-
TRIES LTD**
**1-2 Doshomachi-3-chome**
**Chuo-ku Osaka(JP)**

(72) Inventor: **Tsuchiya, Masakazu**
**60, Gyokicho-4-chome**
**Itami-shi(JP)**
Inventor: **Oishi, Haruki**
**1-1-1107, Kurehacho**
**Ikeda-shi(JP)**
Inventor: **Takaoka, Aya**
**1-5, Omachi**
**Ako-shi(JP)**
Inventor: **Matsuura, Shuji**
**2-E-1, Midoridai-7-chome**
**Kawanishi-shi(JP)**

(74) Representative: **Baillie, Iain Cameron et al**
**c/o Ladas & Parry**
**Altheimer Eck 2**
**D-80331 München (DE)**

## Description

BACKGROUND OF THE INVENTION

This invention relates to a process for measuring the concentration of endotoxin (hereinafter abbreviated as "ET") in a test sample by measuring an optical change due to a gelation reaction caused by reacting horseshoe crab hemocyte lysate (hereinafter abbreviated as "AL solution") with ET, which process can be employed even when there is a fear that the test sample may affects the gelation reaction caused by the reaction of AL solution with ET.

ET's are known as typical pyrogens. It is known that when blood, an infusion or an injection, which is contaminated by ET, is injected into a body, the ET produces serious side effects such as fever, shock, etc. Therefore, it is considered indispensable to prevent troubles by measuring the amount of ET in starting materials, starting waters, etc. for infusions, injections and the like, or various preparations which are injected into a living body.

AL solution has a property of being activated by ET to cause gelation reaction, and a simple, low-cost ET detecting method utilizing this property, i.e., the so-called Limulus test, is widely employed in the fields of medical science, pharmacy and microbiology. In recent years, the method of Limulus test has been variously improved, and in place of semi-quantitative method according to gel-clat technique which was employed at the beginning, an optical measuring method has come to be employed generally. As a result, more precise determination has become possible. The optical measuring method comprises noting a turbidity due to a gel formed by the gelation reaction of AL solution with ET, measuring the change of turbidity as an optical change, for example, a change of the amount of transmitted light, and determining ET from the change of the amount of transmitted light. In this method, the ET concentration in a test sample is determined, for example, from a graph showing the calibration relation (a calibration curve) obtained from a time required for the ratio of transmittance to reach a predetermined value (hereinafter abbreviated as "gelation time") and ET concentration.

However, although the above measuring method employed at present can be employed without a particular trouble for measuring the concentration of ET contained in water itself such as starting water, wash water, etc. or for measuring the ET concentration in a test sample which does not affect the gelation reaction, it is inapplicable to a test sample which has an inhibitory or enhancement effect on the gelation reaction. In detail, in the case of a test sample which inhibits the gelation reaction, the gelation time is increased, resulting in a measured value lower than a real value. In the case of a test sample which enhances the gelation reaction, the gelation time is reduced, resulting in a measured value higher than a real value. In such cases, there is usually employed a conventional means which comprises spiking ET to identical preparations of a standard test sample containing no ET, preparing a calibration curve, for example, for the ET concentration in the thus prepared sample and the gelation time, and measuring the ET concentration in a test sample by using the calibration curve. However, it is actually very difficult to obtain a standard test sample containing no ET and therefore the above means is very difficult to practice.

On the other hand, in U.S. Pharmacopeia (USPXXI: ⟨85⟩ BACTERIAL ENDOTOXIN TEST), preparatory testing for confirming that a test sample itself does not affect the gelation reaction, is obligatory in carrying out Limulus test and is described as INHIBITION OR ENHANCEMENT TEST.

For measuring the ET concentration in a test sample which affects the gelation reaction, there has heretofore been generally employed a dilution method in which the test sample is diluted until its influence is lost. This method, however, involves the following problems. A great deal of labor is required for determining the degree of dilution. Furthermore, in the case of a test sample which requires a high degree of dilution, when ET is not detectable, it is often impossible to judge sufficiently whether the degree of dilution is too high or the amount of ET present in the test sample itself is smaller than a standard value.

On the other hand, as a process for measuring the ET concentration in a test sample which affects the gelation reaction, there can be exemplified the process disclosed in Japanese Patent Appln. Kokai (Laid-Open) No. 62-110153 which was invented by some of the present inventors. In this process, the amount of ET in a test sample is measured using a calibration curve showing the relationship between ET concentration and gelation time which is obtained using, as samples, solutions of predetermined amounts of ET spiked in distilled water (hereinafter abbreviated as "water calibration curve"), and a calibration curve showing the relationship between ET concentration and gelation time which is prepared using three or more samples prepared by spiking predetermined amounts of ET to identical preparations of a test sample (hereinafter abbreviated as "test sample calibration curve"). This process utilizes the following fact found by some of the present inventors: the calibration curve showing the relationship between ET concentration and

2

gelation time which is obtained using samples prepared by spiking predetermined amount of ET to identical preparations of a test sample containing no ET originally, coincides with the water calibration curve when the test sample does not affect the gelation reaction at all; the former is above the latter when the test sample inhibits the gelation reaction; the former is below the latter when the test sample enhances the gelation reaction; and in most of the cases, the former calibration curve is originally parallel to the water calibration curve. In other words, the above fact is utilized as follows: the reason why in actual measurement, the test sample calibration curve has a portion not parallel to the water calibration curve is that the test sample calibration curve undergoes some influence of ET originally contained in the test sample, and therefore the ET content of the test sample can be measured by comparing the test sample calibration curve with the water calibration curve. However, in this process, the ET content of the test sample cannot be measured unless as samples prepared by spiking ET to identical preparations of test sample, there are prepared at least one sample containing spiked ET in an amount substantially equal to or smaller than the amount of ET originally present in the test sample, and two or more samples containing spiked ET in such amounts that the amount of ET originally present in the test sample can be neglected. Accordingly, said process is disadvantageous, for example, in that the ET content of the test sample should be predicted to a certain degree and that procedures such as the preparation of the samples are troublesome. Thus, further improvement of said process is desirable.

SUMMARY OF THE INVENTION

This invention was made in consideration of such conditions and is intended to provide a process which permits simple and rapid measurement of the ET concentration in a test sample which tends to affect the gelation of AL solution by ET.

This invention provides a process for measuring an endotoxin concentration in a test sample by measuring optical changes due to a gelation reaction caused by a reaction of a horseshoe crab hemocyte lysate with endotoxin, which comprises

i) preparing three or more endotoxin spiked samples by spiking endotoxin in known different concentrations to a test sample,

measuring optical changes due to a gelation reaction caused by addition of the horseshoe crab hemocyte lysate to individual endotoxin spiked samples, and

measuring a gelation time required for the optical change to reach a predetermined proportion,

ii) preparing three or more endotoxin spiked solutions by spiking endotoxin in known different concentrations to a solution containing no endotoxin and having no influence on the gelation reaction,

measuring the gelation time in the same manner as mentioned in above i), and

preparing a calibration curve showing a relationship between the gelation time and the spiked endotoxin concentrations,

iii) obtaining apparent endotoxin concentrations in the endotoxin spiked samples from the gelation times obtained in above i) applied on the calibration curve obtained in above ii), and

iv) determining the endotoxin concentration in the test sample by confirming a linearity between the apparent endotoxin concentrations obtained in above iii) and actual spiked endotoxin concentrations used in above i), and using a statistical straight-line test

This invention also provides an apparatus for measuring such ET concentrations.

BRIEF DESCRIPTION OF THE DRAWINGS

Fig. 1 shows a calibration curve obtained in Example 1.

Fig. 2 is a graph showing the relationship between the concentration of spiked ET ($E_s$) and apparent ET concentration ($E_{app}$) which was obtained in Example 1.

Fig. 3 is a graph showing the relationship between $E_s$ and $E_{app}$ which was obtained by use of a dextran solution as sample in Example 2.

Fig. 4 is a graph showing the relationship between $E_s$ and $E_{app}$ which was obtained by use of 20-fold diluted GIT medium in Example 3.

Fig. 5 is a perspective view of outer appearance of one example of the apparatus of this invention.

Fig. 6 is a block diagram showing an electric system of one example of the apparatus of this invention.

## DESCRIPTION OF THE PREFERRED EMBODIMENTS

According to this invention,

(i) three or more ET spiked samples different in ET concentrations are prepared by spiking ET to substantially identical preparations of a test sample, an optical change due to a gelation reaction caused by addition of AL solution to each sample is measured, and a time required for the optical change to reach a predetermined proportion (hereinafter abbreviated as "gelation time") is measured;

(ii) a gelation time is measured in the same manner as in (i) above except for using three or more ET spiked solutions prepared by spiking ET in different concentrations to substantially identical preparations of a solution which neither contains ET nor affects a gelation reaction caused by reacting AL solution with ET, and a calibration curve showing the relationship between the gelation time and the concentration of spiked ET is prepared;

(iii) the apparent ET concentration in each ET spiked sample prepared in (i) above is calculated by extrapolating each gelation time measured in (i) above to the calibration curve obtained in (ii) above; and

(iv) the concentrations of ET actually spiked to the preparations of test sample in (i) above and the corresponding apparent ET concentrations measured in (iii) above are subjected to statistical processing to judge whether a linearity exists between the former concentrations and the latter concentrations, and after confirming the existence of the linearity, the ET concentration in the test sample is easily determined from the values of the intercept and the slope of a regression line formula obtained by the statistical processing.

This invention is conducted, for example, as follows.

First, at least three ET spiked solutions different in ET concentrations are prepared by spiking ET in various concentrations to substantially identical preparations of a solution which neither contains ET nor affects a gelation reaction caused by reacting AL solution with ET, and each of them is reacted with AL solution, after which a calibration curve showing the relationship between ET concentration and gelation time (hereinafter abbreviated as "ET calibration curve") is prepared. Subsequently, at least three ET spiked samples different in ET concentrations are prepared by spiking ET to substantially identical preparations of a test sample in various concentrations and each of them is reacted with AL solution, after which the gelation time of each of them is measured. From this gelation time and the previously prepared ET calibration curve, the apparent ET concentration ($E_{app}$) of each ET spiked sample prepared from the preparation of test sample is determined. Using a conventional method of statistical straight-line test, for example, a method comprising processing $E_{app}$ and the concentration of actually spiked ET ($E_s$) statistically and determining a correlation coefficient ($\gamma$), there is judged whether or not the assumption that the influence of the test sample on the gelation reaction is brought about in a constant proportion in the range of ET concentration measured, is good; in other words, whether or not a linearity exists between $E_{app}$ and $E_s$; namely, whether or not the calibration curve showing the relationship between the concentration of spiked ET and gelation time, prepared on the basis of the results obtained by reacting each ET spiked sample prepared by spiking a predetermined amount of ET to the preparation of test sample with AL solution, is parallel to the ET calibration curve. When the existence of the linearity is confirmed, there is set up the linear relationship formula:

$$E_{app} = a + b \times E_s \qquad (1)$$

The coefficients "a" and "b" of this linear relationship formula can be interpreted as follows.

When the potency appearance ratio of ET in the sample prepared from the preparation of test sample (a value obtained by dividing $E_{app}$ by the real ET concentration in said sample) is taken as R and the real ET concentration in said sample as E, $E_{app}$ is given by

$$E_{app} = R \times E \qquad (2)$$

Since E is the sum of the concentration of ET originally contained in the test sample ($E_c$) and $E_s$, the equation (2) becomes

$$E_{app} = R \times (E_c + E_s)$$

so that

$$E_{app} = R \times E_c + R \times E_s \qquad (3)$$

4

Comparison of the equation (3) with the equation (1) gives

$$a = R \times E_c \quad (4)$$

$$b = R \quad (5)$$

That is, the potency appearance ration R is expressed as "b", and the influence on the gelation reaction caused in the test sample by the reaction of AL solution with ET is judged as follows depending on a value which R takes:

R ≒ 1 : not affected by the test sample

R < 1 : inhibited by the test sample

R > 1 : enhanced by the test sample

From the above equations (4) and (5), it can be seen that the concentration of ET originally contained in the test sample, i.e., $E_c$, can be calculated by dividing "a" by "b".

In the present invention, in order to measure the real ET concentration ($E_c$) in the test sample, suitable amounts of ET are spiked to the test sample and the solution containing no ET. The amounts (or concentration) of ET to be spiked are not particularly limited but preferably selected from the range of 0.2 to 20 times, more preferably 0.5 to 10 times, as large as the predetermined endotoxin concentration for judgement depending on purposes ($E_{det}$). At least three known different concentrations of ET are spiked to each test sample and each solution containing no ET to give at least 6 sample solutions to be measured. In this case, when $E_c$ and $E_{det}$ are quite different, there is a tendency to lower reliability of $E_c$ measured. But, even in such a case, since it is possible to surely judge whether $E_c$ is larger or smaller than $E_{det}$, there arises no problem practically. In order to obtain more precise values, it is desirable to spike at least three different concentrations selected from the range of 0.2 to 20 times, more preferably 0.5 to 10 times, as large as the real ET concentration to each test sample and each solution containing no ET. By this, the linear relationship formula (1) mentioned above is established. Further, in order to prepare three ET solutions to be spiked and having different concentrations, a so-called 2-fold dilution method is preferably employed.

In this invention, the optical change to reach a predetermined proportion ($R_{th}$) is provided in the range of 75 to 97% in terms of R(t). In this case, R(t) is defined as follow: when an initial transmittance amount on a mixture of AL solution and ET spiked sample (or ET spiked solution) is $I_o$ and a transmittance amount measured with the laspe of time on the mixture is I(t), $R(t) = I(t)/I_o \times 100$.

When $R_{th}$ is provided at lower than 75%, for example, $R_{th} = 70\%$, it becomes very difficult to detect the gelation and no calibration curve is obtained, or if obtained the range of calibration curve is very narrrowed to 0.1 to 10 ng/ml. On the other hand, when $R_{th}$ is provided at higher than 97%, the moving (convection) in the mixture immediately after mixing the AL solution with ET spiked sample or ET spiked solution, or the variation of R(t) due to the movement of bubbles is possibly judged as gelation by mistake. Therefore, in order to prevent an erroneous detection due to such noises, the range of 3% is necessary for allowance of noise.

That is, $R_{th}$ is provided in the range of 75 to 97%, preferably in the range of 80 to 95%.

Needless to say, the process of this invention is applicable not only to a test sample which is clearly known to affect the gelation reaction but also to a test sample which is not clearly known to affect and a test sample which does not affect.

Although the solution containing no ET and having no influence on the gelation reaction caused by reacting AL solution with ET which is used for preparing the calibration curve, is not critical so long as it has these properties, pyrogen-free distilled water, more specifically, distilled water for injection, is usually preferably used, for example, because it is easily available.

The AL solution usable in the process of this invention is not critical so long as it can be used for usual measurement of ET. There may be used, for example, AL solutions prepared from freeze-dried products of AL solutions which are commercially available, for instance, from Associates of Cape Cod Inc. (ACC), HAEMACHEM, Inc., Whittaker Bioproducts, Inc, and Teikoku Hormon Mfg. Co., Ltd. In addition, there can be exemplified any AL solution without particular restriction so long as it is extracted from hemocytes of horseshoe crab belonging to Limulus genus, Tachypleus genus or Carcinoscorpius genus and reacts with ET to undergo gelation reaction.

Although needless to say, the measuring process of this invention can easily be practiced by means of an apparatus exclusively for kinetic turbidimetric technique such as Toxinometer ET-201 (mfd. by Wako Pure Chemical Industries, Ltd.), LAL-5000 (mfd. by ACC Inc.), etc., it can be practiced also by means of a measuring apparatus utilizing an optical principle, such as MS-2 of Abbot Laboratries, etc.

For practicing the process of this invention, it is more convenient to utilize, for example, a microcomputer incorporated with a software which permits examination of linear relationship and calculation of the ET concentration in a test sample by input of obtained values of $E_{app}$ and $E_s$.

The present invention also provides an apparatus for measuring endotoxin comprising:

(a) a constant temperature bath for standing at least one sample cuvette containing a mixture of a sample and a horseshoe crab hemocyte lysate (AL) (a reaction solution) at a constant temperature state,

(b) a means for irradiating a light on individual sample cuvettes maintained at a constant temperature state and detecting transmitted light amount in series,

(c) a means for indicating the beginning of measurement of transmitted light amount through said reaction solution,

(d) a means for measuring a gelation time required for the transmitted light amount change to reach a predetermined proportion from the beginning of the measurement,

(e) a means for obtaining an equation showing a relationship between the gelation time obtained in (d) and the endotoxin concentration as to three or more samples having known endotoxin concentrations with different concentrations, respectively, and a means for storing said equation,

(f) a means for obtaining an apparent endotoxin concentration by inserting the gelation time obtained in (d) for three or more samples adjusted by spiking different known amounts of endotoxin thereto into the equation obtained in (e),

(g) a means for detecting linearity between the spiked endotoxin concentrations and the apparent endotoxin concentrations obtained in (f) for individual samples used in (f) and a means for obtaining a linear relationship equation between both concentrations, and

(h) a means for obtaining an endotoxin concentration in the test sample from the slope and an intercept obtained in (g).

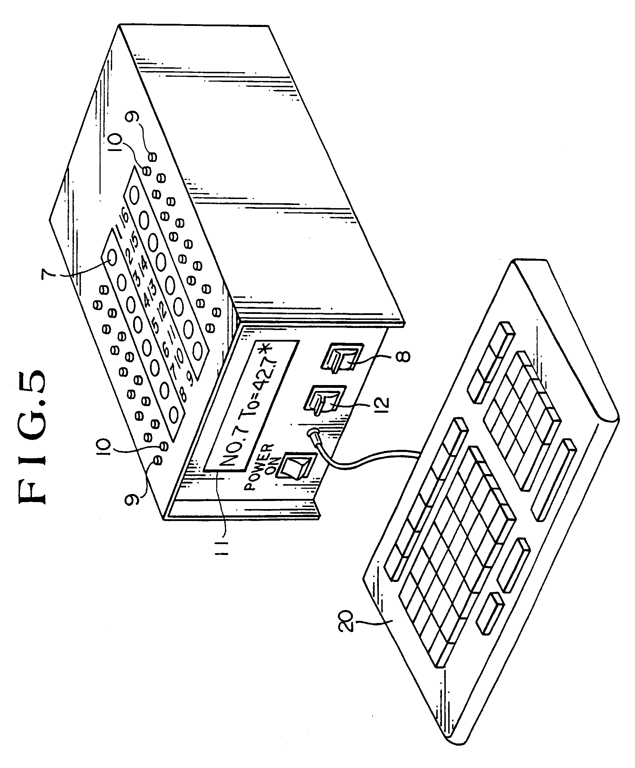

One concrete example of an apparatus according to this invention is shown in Fig. 5. In Fig. 5, a plurality of cuvette holders 7 for holding cuvettes for measurement are provided in one apparatus. Each one optical system for measuring transmitted light amounts is provided in one holder 7. It is also possible to apply a light to each holder by using an optical fiber from one light source. Further, all the holders are kept at a constant temperature such as at 37°C by a constant temperature bath (not shown in Fig. 5) or a dry temperature controlling apparatus. After fixing each cuvette containing the reaction solution therein in a holder, the reaction solution can be maintained at a still state until the end of the measurement and the measurement can be conducted without giving any moving so as to break the gel state. After fixing a cuvette for measurement containing a mixture of a liquid sample and the LAL reagent in a cuvette holder 7, a switch 8 for instructing the beginning of the measurement is instantly pushed to begin the measurement of the sample.

Then, the switch 8 is pushed every time when a cuvette for measurement is fixed in a holder 7 in a similar manner to conduct the parallel measurement of a plurality of samples with an optional timing within the number of cuvette holders provided in the apparatus. Fig. 5 shows that it is possible to fix 16 cuvettes in the holders in the apparatus. But the number of cuvettes measurable at a time is not limited to such a number of 16 and can be increased or decreased if desired so long as the capacity for control is permissible. It is also possible to connect switches for instructing the beginning of measurement to each cuvette holder 7. It is further possible to automatically switch on when each cuvette is placed in each cuvette holder so as to instruct the beginning of measurement.

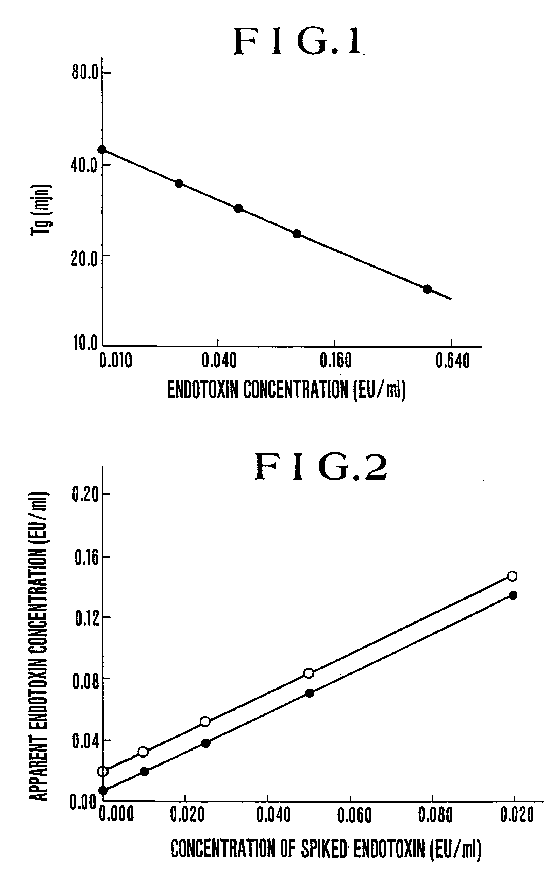

It is possible to install a number of measurement display LED (light-emitting diode) 9 for displaying the state of corresponding optical systems. By such displays, it is possible to know which optical system is to be measured from the time of pushing the switch 8 or which optical system is in the course of measurement. Further, when a number of gelation judgement display LED 10 are installed in the apparatus, the judgement of positive/negative for each sample can be displayed. Thus, the same results as obtained by the prior art method of judging by the naked eye can be displayed objectively with high precision in a short time. In addition, when the gelation time ($T_g$) based on the gelation judgement of each optical system is displayed by display panel 11 which can display letters and figures, the determination of endotoxin concentration becomes possible from the relation between the endotoxin concentration and $T_g$ as shown in Fig. 1 rapidly with good reproducibility in addition to the positive/negative judgement.

A display control switch 12 can be used for changing one after another the display of each $T_g$ of each sample when a plurality of samples are measured. It is also possible to display as a whole by using another display method such as CRT display.

A keyboard 20 connected to the main body of the apparatus is used for appointment of operational function, input of numeral data, and the like. When data are input, the display panel 11 is also used for confirming display of input data.

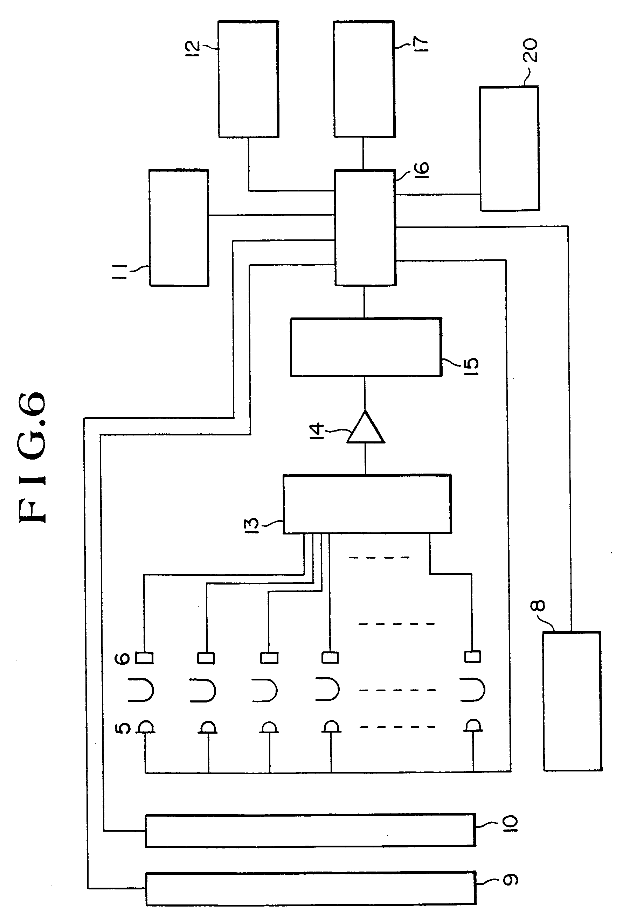

Fig. 6 is a block diagram showing an electric system of one example of the apparatus of this invention. In Fig. 6, the constant temperature controlling system around the cuvettes for measurement is omitted for simplicity and only the optical measurement controlling system is shown. The transmitted light amounts obtained from a plurality of photoelectric detectors 6 are selected one after another successively by a multiplexer 13, converted to R(t) by a ration operating circuit 14, and input into an A/D converter 15. The signal converted to digital amount by the A/D converter is processed by a computer 16 to give the judgement of gelation and the determination of $T_g$. The gelation judgement display 10 and the display panel 11 are also controlled by the computer.

The computer 16 prepares and stores calibration curves relating to the gelation time and the ET concentration from the measuring results regarding samples containing ET in known concentrations based on the data input from the keyboard 20. Further, by properly using programs in the computer 16, it is possible to obtain apparent ET concentrations in samples from the gelation times of the samples to which ET is spiked using said calibration curves, to detect linearity between the spiked ET concentrations and the apparent ET concentrations so as to obtain an equation of linear relationship between both concentrations, and to obtain the ET concentration in the sample from a slope and an intercept of the equation of linear relationship. It is also possible to print out the measurement results and the data processing results by a printer 17. It is still possible to connect the computer 16 to one or more outer computers, if required, depending on purposes.

According to this invention, it is not necessary to use specially shaped cuvettes for the measurements and there can be used ordinary cuvettes used for a prior art manual method.

As mentioned above, this invention provides an apparatus for measuring endotoxin in a plurality of samples in parallel and effectively with high reliability. Further, by using the apparatus of the present invention, the measurement can be carried out precisely even when a test sample includes substances which may affect the gelation reaction caused by the reaction of AL solution with ET.

This invention is more concretely explained below with reference to Examples and reference Examples, which are not by way of limitation but by way of illustration.

Example 1

[Reagents]

• ET solutions

There were used solutions prepared by diluting Escherichia coli control standard endotoxin (a lipopolysaccharide derived from E. coli UKT-B strain, available from Wako Pure Chemical Industries, Ltd.; each vial contained the lipopolysaccharide in an amount corresponding to 500 ng of FDA reference standard endotoxin EC-2; for dissolution in 5 ml) properly with distilled water for injection.

• AL solution

A freeze-dried product of AL solution derived from horseshoe crab belonging to Limulus genus (hereinafter the freeze-dried product being abbreviated as "LAL"; available from Associated of Cape Cod, Inc.; labeled sensitivity 0.03 EU/ml; for dissolution in 5 ml) was dissolved in 5 ml of distilled water for injection, and the LAL solution thus obtained was used as AL solution.

[Samples]

i) Group I

Solutions having a sorbitol concentration of 2% and a concentration of spiked ET of 0.01, 0.025, 0.05 or 0.1 EU/ml, respectively, were prepared as samples by dissolving sorbitol (mfd. by Wako Pure Chemical Industries, Ltd.; guaranteed reagent) in distilled water for injection to a concentration of 2.2%, followed by adding thereto each of ET solutions having predetermined concentrations.

ii) Group II

Solutions having a sorbitol concentration of 2% and a concentration of spiked ET of 0.01, 0.025, 0.05 or 0.1 EU/ml, respectively, were prepared as samples by dissolving the same sorbitol as used in i) above in ET solution (0.011 EU/ml) to a concentration of 2.2%, followed by adding thereto each of ET solutions having predetermined concentrations.

[Measuring procedure]

Measurement was carried out as follows by means of a Toxinometer ET-201 (mfd. by Wako Pure Chemical Industries, Ltd.) according to a conventional method.

To 0.1 ml of the LAL solution was added 0.1 ml of each sample, and after stirring, a time required for reducing the transmittance by 5% (hereinafter abbreviated as "$T_g$") was measured while maintaining the temperature at 37°C.

Separately, the same measurement as described above was carried out except for using as samples, ET solutions various in their concentrations prepared from distilled water for injection and the predetermined ET solution, and a calibration curve showing the relationship between ET concentration and $T_g$ (hereinafter abbreviated as "water calibration curve") was prepared.

[Results]

The water calibration curve obtained in the above is shown in Fig. 1.

In Table 1 are shown the results of determining the apparent ET concentration ($E_{app}$) of each solution of Group 1 by using the water calibration curve. In Table 1, $E_s$ denotes the concentration of spiked ET, and each value of $E_{app}$ is the average of three measurements.

Table 1

| $E_s$ (EU/ml) | $E_{app}$ (EU/ml) |
|---|---|
| 0.010 | 0.020 |
| 0.025 | 0.040 |
| 0.050 | 0.069 |
| 0.100 | 0.138 |

In Fig. 2, the relationship between the $E_{app}$ value and the corresponding $E_s$ value which was determined for each sample of Group 1 is shown by -●-. The straight line -●- in Fig. 2 is a regression line obtained according to individual data by the least squares method.

As is clear from the above results, a satisfactory correlation (linearity) (correlation coefficient $\gamma$ = 0.999) existed between the $E_{app}$ values obtained for the samples of Group I and the corresponding $E_s$ values and it was found that the water calibration curve was highly parallel to a calibration curve showing the relationship between ET concentration and $T_g$ which was obtained using the samples of Group I.

A regression line formula obtained by processing the above $E_s$ and $E_{app}$ values statistically was

$$E_{app} = 1.307 \times E_s + 0.006$$

Thus, the potency appearance ratio R was 1.307, and the ET concentration in the 2% sorbitol solution of Group I used in this case was calculated as follows:

$$0.006 \div 1.307 = 0.005 \text{ EU/ml}$$

In Fig. 2, the relationship between the $E_{app}$ value and the corresponding $E_s$ value which was determined for each sample of Group II is shown by -o-. The straight line -o- in Fig. 2 is a regression line obtained according to individual data by the least squares method.

As is clear from the above results, a satisfactory correlation (linearity) (correlation coefficient $\gamma$ = 1.000) existed also between the $E_{app}$ values obtained for the samples of Group II and the corresponding $E_s$ values, and it was found that the water calibration curve was highly paralled also to a calibration curve showing the relationship between ET concentration and $T_g$ which was obtained using the samples of Group II.

8

A regression line formula obtained by statistical processing of the $E_{app}$ values obtained for the samples of Group II and the corresponding $E_s$ values was

$$E_{app} = 1.270 \times E_s + 0.021$$

Thus, the potency appearance ratio R was 1.270, and the ET concentration in the 2% sorbitol solution of Group II used in this case was calculated as follows:

$$0.021 \div 1.270 = 0.017 \text{ EU/ml}$$

The difference between the concentration of ET detected in the 2% sorbitol solution of Group II and the concentration of ET detected in the 2% sorbitol solution of Group I is 0.012 EU/ml, which is in substantial agreement with the difference between the ET concentrations in the two 2.2% sorbitol solutions used for preparing the 2% sorbitol solution. This fact indicates that the assumption made in this invention is valid.

Example 2

[Reagents]

• ET solutions

The same ET solutions as used in Example 1 were used.

• AL solution

There was used a LAL solution obtained by dissolving LAL (available from Associated of Cope Cod. Inc.; labeled sensitivity 0.25 EU/ml; for dissolution in 5 ml) in 5 ml of distilled water for injection.

[Samples]

Solutions having a dextran concentration of 5% and a concentration of spiked ET of 0.05, 0.2 or 2.0 EU/ml were prepared as samples by dissolving dextran (available from Wako Pure Chemical Industries, Ltd.; molecular weight 100,000 to 200,000) in distilled water for injection to a concentration of 5.5%, followed by adding thereto each of ET solutions having predetermined concentrations.

[Measuring procedure]

Measurement was carried out by the same procedure as in Example 1.

[Results]

In Table 2 are shown the results of determining the apparent ET concentration ($E_{app}$) of each sample by using the water calibration curve prepared in Example 1. In Table 2, $E_s$ denotes the concentration of spiked ET, and each value of $E_{app}$ is the average of three measurements.

Table 2

| $E_s$ (EU/ml) | $E_{app}$ (EU/ml) |
|---|---|
| 0.050 | 0.428 |
| 0.200 | 1.062 |
| 2.000 | 6.592 |

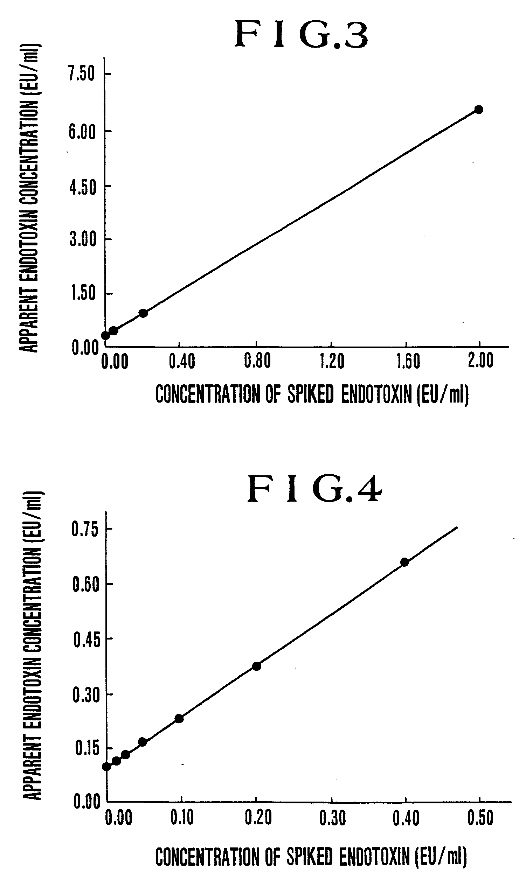

The relationship between the $E_{app}$ values obtained and the corresponding $E_s$ values is shown in Fig. 3. The straight line in Fig. 3 is a regression line obtained according to individual data by the least squares method.

As is clear from the above results, a satisfactory correlation (linearity) (correlation coefficient $\gamma = 1.000$) existed between $E_s$ and $E_{app}$ also when the dextran solutions were used as samples, and it was found that the water calibration curve was highly parallel to a calibration curve obtained for ET concentration vs. $T_g$ by the measurement using the dextran solutions as samples.

A regression line formula obtained by processing the above $E_s$ and $E_{app}$ values statistically was

$$E_{app} = 3.124 \times E_s + 0.351$$

Thus, the potency appearance ratio R was 3.124, and the ET concentration in the 5% dextran solution used in this case was calculated as follows:

$$0.351 \div 3.124 = 0.112 \text{ EU/ml}$$

Example 3

[Reagents]

• ET solutions

The same ET solutions as used in Example 1 were used.

• AL solution

There was used a LAL solution obtained by dissolving LAL (available from Wako Pure Chemical Industries, Ltd.; labeled sensitivity 0.03 EU/ml; for dissolution in 5 ml) in 5 ml of distilled water for injection.

[Samples]

Solutions having a concentration of added ET of 0.0125, 0.025, 0.050, 0.10, 0.20 or 0.40 EU/ml, respectively, were prepared as samples by diluting GIT medium (a synthetic medium for cell culture, mfd. by Wako Pure Chemical Industries, Ltd.) in 20 times with distilled water for injection, and adding 0.1 ml of each of ET solutions having predetermined concentrations to 0.9 ml of the diluted medium.

[Measuring procedure]

Measurement was carried out by the same procedure as in Example 1.

[Results]

In Table 3 are shown the results of determining the apparent ET concentration ($E_{app}$) of each sample by use of the water calibration curve prepared in Example 1. In Table 3, $E_s$ denotes the concentration of spiked ET, and each value of $E_{app}$ is the average of three measurements.

Table 3

| $E_s$ (EU/ml) | $E_{app}$ (EU/ml) |
|---|---|
| 0.0125 | 0.1177 |
| 0.0250 | 0.1256 |
| 0.0500 | 0.1651 |
| 0.1000 | 0.2545 |
| 0.2000 | 0.3744 |
| 0.4000 | 0.6578 |

The relationship between the $E_{app}$ values obtained and the corresponding $E_s$ values is shown in Fig. 4. The straight line in Fig. 4 is a regression line obtained by the least squares method.

As is clear from the above results, a satisfactory correlation (linearity) (correlation coefficient $\gamma = 0.999$) existed between $E_s$ and $E_{app}$ also when the GIT medium solutions were used as samples, and it was found that the water calibration curve was highly parallel to a calibration curve obtained for ET concentration vs. $T_g$ by the measurement using the GIT medium solutions as samples.

A regression line formula obtained by processing the above $E_s$ and $E_{app}$ values statistically was

$$E_{app} = 1.399 \times E_s + 0.099$$

Thus, the activity appearance ratio R was 1.399, and the ET concentration in the about 20-fold diluted GIT medium used in this case was calculated as follows:

$$0.099 \div 1.399 = 0.071 \text{ EU/ml}$$

As described above, this invention provides a process by which the ET concentration in a test sample which tends to affect the gelation reaction of AL solution by ET can be measured easily and rapidly without a troublesome procedure required in conventional methods.

**Claims**

1. A process for measuring an endotoxin concentration in a test sample by measuring optical changes due to a gelatin reaction caused by a reaction of a horseshoe crab hemocyte lysate with endotoxin, which comprises
   i) preparing three or more endotoxin spiked samples by spiking endotoxin in known different concentrations to a test sample,
   measuring optical changes due to a gelatin reaction caused by addition of the horseshoe crab hemocyte lysate to individual endotoxin spiked samples, and
   measuring a gelation time required for the optical change to reach a predetermined proportion,
   ii) preparing three or more endotoxin spiked solutions by spiking endotoxin in known different concentrations to a solution containing no endotoxin and having no influence on the gelation reaction,
   measuring the gelation time in the same manner as mentioned in above i), and
   preparing a calibration curve showing a relationship between the gelation time and the spiked endotoxin concentrations,
   iii) obtaining apparent endotoxin concentrations in the endotoxin spiked samples from the gelation times obtained above i) applied on the calibration curve obtained in above ii), and
   iv) determining the endotoxin concentration in the test sample by confirming a linearity between the apparent endotoxin concentrations obtained in above iii) and actually spiked endotoxin concentrations used in above i), and using a statistical straight-line test.

2. A process according to claim 1, wherein the solution to which endotoxin is to be spiked is distilled water for injection.

3. A process according to claim 1, wherein the concentration of endotoxin to be spiked to the test samples or the solutions is in the range of 0.2 to 20 times as large as the predetermined endotoxin concentration for judgement depending on purposes.

4. A process according to claim 1, wherein the statistical straight-line test comprises setting up a linear relationship formula:

$$E_{app} = a + b \times E_s$$

wherein $E_{app}$ is an apparent endotoxin concentration; $E_s$ is a concentration of endotoxin actually spiked; and "a" and "b" are coefficient, and calculating the value of a/b.

5. An apparatus for measuring endotoxin comprising
   (a) a constant temperature bath for standing at least one sample cuvette containing a mixture of a test sample and a horseshoe crab hemocyte lysate at a constant temperature state,
   (b) a means for irradiating a light on individual sample cuvettes maintained at a constant temperature state and detecting transmitted light amount in series,
   (c) a means for indicating the beginning of measurement of transmitted light amount through said reaction solution,

EP 0 350 273 B1

(d) a means for measuring a gelation time required for the transmitted light amount change to reach a predetermined proportion from the beginning of the measurement,

(e) a means for obtaining an equation showing a relationship between the gelation time obtained in (d) and the endotoxin concentration as to three or more samples having known endotoxin concentrations with different concentrations, respectively, and a means for storing said equation,

(f) a means for obtaining an apparent endotoxin concentration by inserting the gelation time obtained in (d) for three or more samples adjusted by spiking different known amounts of endotoxin thereto into the equation obtained in (e),

(g) a means for detecting linearity between the spiked endotoxin concentrations and the apparent endotoxin concentrations obtained in (f) for individual samples used in (f) and a means for obtaining a linear relationship equation between both concentrations, and

(h) a means for obtaining an endotoxin concentration in the test sample from the slope and an intercept obtained in (g).

**Patentansprüche**

1. Verfahren zum Messen einer Endotoxinkonzentration in einer Probe durch Messen von optischen Veränderungen, die auf eine Gelierreaktion zurückzuführen sind, die durch eine Reaktion eines Hämozytenlysats von Königskrabben mit Endotoxin verursacht wird, in dem

   i) zum Herstellen von drei oder mehr mit Endotoxin versetzten Proben eine Testprobe in verschiedenen bekannten Konzentrationen mit Endotoxin versetzt wird,

   optische Veränderungen gemessen werden, die auf eine Gelierreaktion zurückzuführen sind, die durch Zugabe des Hämozytenlysats von Königskrabben zu einzelnen mit Endotoxin versetzten Proben herbeigeführt worden ist, und

   die Gelierzeit gemessen wird, die verstreicht, bis die optische Veränderung ein vorherbestimmtes proportionales Ausmaß erreicht,

   ii) zum Herstellen von drei oder mehr mit Endotoxin versetzten Lösungen eine Lösung, die kein Endotoxin enthält, und keinen Einfluß auf die Gelierreaktion hat, in verschiedenen bekannten Konzentrationen mit Endotoxin versetzt wird,

   die Gelierzeit,wie vorstehend in i) beschrieben, gemessen wird und

   eine Eichkurve gezeichnet wird, die die Beziehung zwischen der Gelierzeit und den Konzentrationen des Endotoxins in den damit versetzten Proben darstellt,

   iii) mit Hilfe der vorstehend nach ii) erhaltenen Eichkurve auf Grund der Gelierzeiten die scheinbaren Konzentrationen des Endotoxins in den damit versetzten Proben bestimmt werden und

   iv) zum Bestimmen der Endotoxinkonzentration in einer Testprobe in einem statistischen Geradlinigkeitstest kontrolliert wird, ob zwischen den nach iii) ermittelten, scheinbaren Endotoxinkonzentrationen und den nach i) verwendeten tatsächlichen Konzentrationen des Endotoxins in den damit versetzten Proben ein linearer Zusammenhang besteht.

2. Verfahren nach Anspruch 1, in dem das mit dem Endotoxin zu versetzende Lösung destilliertes Wasser für Injektionen ist.

3. Verfahren nach Anspruch 1, in dem für eine Beurteilung je nach dem beabsichtigten Zweck die Konzentration, in der die Testproben oder die Lösungen mit Endotoxin versetzt werden sollen, im Bereich des 0,2- bis 20-fachen der vorherbestimmten Endotoxinkonzentration liegt.

4. Verfahren nach Anspruch 1, in dem in dem statistischen Geradlinigkeitstest eine Formel

$$E_{pp} = a + b \times E_s$$

für eine lineare Beziehung aufgestellt wird, in der $E_{app}$ eine scheinbare Endotoxinkonzentration, $E_s$ eine tatsächliche Konzentration von Endotoxin in einer damit versetzten Probe ist, "a" und "b" Koeffizienten sind, und der Wert a/b berechnet wird.

5. Vorrichtung zum Messen von Endotoxin mit

   (a) einem Konstanttemperaturbad zur Aufnahme mindestens einer Probenküvette, die geeignet ist, ein Gemisch einer Testprobe und einem Hämozytenlysat von Königskrabben auf einer konstanten Temperatur zu halten,

(b) einer Einrichtung zum Bestrahlen einzelner auf einer konstanten Temperatur gehaltenen Probenküvetten und zum Erfassen der hindurchgetretenen Lichtmengen,

(c) einer Einrichtung zum Anzeigen des Beginns der Messung der durch die Reaktionslösung hindurchgetretenen Lichtmenge

(d) einer Einrichtung zum Messen der Gelierzeit, die nach dem Beginn der Messung verstreicht, bis die Veränderung der Menge des hindurchgetretenen Lichts einen vorherbestimmten anteiligen Betrag erreicht,

(e) einer Einrichtung zum Ermitteln einer Gleichung, die die Beziehung zwischen der nach (d) ermittelten Gelierzeit und der Endotoxinkonzentration von drei oder mehr Proben besteht, die bekannte, unterschiedliche Endotoxinkonzentrationen haben, und einer Einrichtung zum Speichern dieser Gleichung,

(f) einer Einrichtung zum Ermitteln einer scheinbaren Endotoxinkonzentration durch Einsetzen der in (d) ermittelten Gelierzeit und der Endotoxinkonzentration für drei oder mehr durch Versetzen mit Endotoxin in verschiedenen bekannten Mengen eingestellten Proben in die nach (e) ermittelte Gleichung,

(g) einer Einrichtung zum Feststellen einer linearen Beziehung zwischen den Konzentrationen von Endotoxin in den damit versetzten Proben und den für einzelne nach (f) verwendete Proben ermittelten, scheinbaren Endotoxinkonzentrationen, und einer Einrichtung zum Ermitteln einer eine lineare Beziehung zwischen beiden Konzentrationen darstellenden Gleichung und

(h) einer Einrichtung zum Ermitteln einer Endotoxinkonzentration in der Testprobe auf Grund der Neigung und eines Schnittpunktes, die in (g) ermittelt wurden.

**Revendications**

1. Procédé pour mesurer la concentration d'endotoxine dans un échantillon expérimental en mesurant les changements optiques dus à une réaction de gélification provoquée par la réaction d'un lysat d'hémocytes de limule avec l'endotoxine, dans lequel

i) on prépare trois échantillons, ou plus, d'endotoxine en injectant de l'endotoxine à différentes concentrations connues , dans un échantillon expérimental,

on mesure les changements optiques dus à une réaction de gélification provoquée par l'addition du lysat d'hémocytes de limule à des échantillons individuels ayant reçu une injection d'endotoxine, et

on mesure le temps de gélification nécessaire pour que le changement optique atteigne une proportion prédéterminée,

ii) on prépare trois ou plusieurs solutions d'endotoxine en injectant de l'endotoxine à différentes concentrations connues, dans une solution ne contenant pas d'endotoxine et n'ayant pas d'influence sur la réaction de gélification,

on mesure le temps de gélification de la même manière que mentionné en i) ci-dessus, et

on prépare une courbe d'étalonnage montrant la relation entre le temps de gélification et les concentrations d'endotoxine introduites par injection,

iii) on obtient les concentrations apparentes d'endotoxine dans les échantillons d'endotoxine à partir des temps de gélification obtenus en i) ci-dessus appliqués à la courbe d'étalonnage obtenue en ii) ci-dessus, et

iv) on détermine la concentration d'endotoxine dans l'échantillon expérimental en confirmant une linéarité entre les concentrations apparentes d'endotoxine obtenues en iii) ci-dessus et les concentrations effectives d'endotoxine introduites par injection utilisées en i) ci-dessus, et en utilisant un test statistique en ligne droite.

2. Procédé selon la revendication 1, dans lequel la solution dans laquelle on doit injecter de l'endotoxine est de l'eau distillée injectable.

3. Procédé selon la revendication 1, dans lequel la concentration d'endotoxine à introduire par injection dans les échantillons expérimentaux ou les solutions est comprise dans un intervalle de 0,2 à 20 fois la concentration prédéterminée d'endotoxine en vue d'une évaluation selon les objectifs recherchés.

4. Procédé selon la revendication 1, dans lequel le test statistique en ligne droite comprend l'établissement d'une formule de relation linéaire:

$$E_{app} = a + b \times E_s$$

où $E_{app}$ est la concentration apparente d'endotoxine; $E_s$ est la concentration d'endotoxine effectivement introduite par injection ; et "a" et "b" sont des coefficients, et le calcul de la valeur de a/b.

5. Appareil pour mesurer l'endotoxine, comprenant

(a) un bain à température constante pour laisser reposer au moins une cuvette d'échantillon contenant un mélange d'un échantillon expérimental et d'un lysat d'hémocytes de limule dans un état de température constante,

(b) un moyen pour irradier de la lumière sur les cuvettes d'échantillon individuelles maintenues dans un état de température constante et détecter la quantité de lumière transmise en série,

(c) un moyen pour indiquer la début de la mesure de la quantité de lumière transmise à travers ladite solution réactionnelle,

(d) un moyen pour mesurer le temps de gélification nécessaire pour que la quantité de lumière transmise se modifie pour atteindre une proportion prédéterminée à partir du début de la mesure,

(e) un moyen pour obtenir une équation montrant la relation entre la temps de gélification obtenu en (d) et la concentration d'endotoxine sous la forme de trois échantillons ou plus ayant des concentrations d'endotoxine connues avec des concentration différentes, respectivement, et un moyen pour emmagasinar ladite équation,

(f) un moyen pour obtenir la concentration apparente d'endotoxine en insérant la temps de gélification obtenu en d) pour trois ou plusieurs échantillons que l'on ajuste en y injectant différentes quantités connues d'endotoxine, dans l'équation obtenue en (e),

(g) un moyen pour détacter une linéarité entre les concentrations d'endotoxine introduites par injection et les concentrations apparentes d'endotoxine obtenues en (f) pour des échantillons individuels utilisés an (f) et un moyen pour obtenir une équation de relation linéaire entra les deux concentrations, et

(h) un moyen pour obtenir la concentration d'endotoxine dans l'échantillon expérimental à partir de la pente et de l'ordonnée à l'origine obtenue en (g).

F I G.1

F I G.2

# F I G.3

# F I G.4

EP 0 350 273 B1

FIG.5

NO.7 To=42.7*

POWER ON

17

# F I G.6

EP 0 350 273 B1